# EUROPEAN PATENT SPECIFICATION

(11) **EP 3 731 829 B1**
(45) Date of publication and mention of the grant of the patent: **17.07.2024**
(21) Application number: 18849451.2
(22) Date of filing: 21.12.2018
(51) Int. Cl.: A61K 9/28, A61K 9/20, A61K 31/00, A61K 31/138, A61K 31/222

(54) **TABLET COMPOSITIONS OF FESOTERODINE FUMARATE**
TABLETTENZUSAMMENSETZUNGEN VON FESOTERODINFUMARAT
COMPOSITIONS DE COMPRIMÉS DE FUMARATE DE FÉSOTÉRODINE

(30) Priority: 25.12.2017 TR 201721437; 17.12.2018 TR 201819578
(43) Date of publication of application: 04.11.2020
(73) Proprietor: Sanovel Ilac Sanayi Ve Ticaret Anonim Sirketi, 34460 Sariyer/Istanbul (TR)
(72) Inventor: PEHLIVAN AKALIN, Nur, 34460 Istanbul (TR); AKKAYA, Kerim, 34460 Istanbul (TR); USLU, Ezgi, 34460 Istanbul (TR)
(74) Representative: Sevinç, Erkan
(86) International application number: PCT/TR2018/050860
(87) International publication number: WO 2019/132832

(56) References cited:
- EP-A1- 2 508 173
- WO-A1-2007/141298
- WO-A1-2014/138603
- US-A1- 2015 182 629
- US-B2- 9 504 654
- S. JOSHI ET AL: "Film coatings for taste masking and moisture protection", INTERNATIONAL JOURNAL OF PHARMACEUTICS, vol. 457, no. 2, 1 December 2013 (2013-12-01), pages 395 - 406, XP055103522, ISSN: 0378-5173, DOI: 10.1016/j.ijpharm.2013.10.021
- "Prolonged Release Tablets of Fesoterodine", RESEARCH DISCLOSURE, KENNETH MASON PUBLICATIONS, HAMPSHIRE, UK, GB, vol. 572, no. 8, 1 December 2011 (2011-12-01), pages 1310, XP007140976, ISSN: 0374-4353

## Description

### Field of the Invention

The present invention relates to extended release tablet compositions comprising fesoterodine fumarate and at least one coating comprising coating agents as defined in claim 1 and the compositions are stable against fesoterodine degradation over an extended period of time. The present invention also relates to a simple, rapid, cost effective, time-saving and industrially convenient method of preparing the extended release tablet compositions of fesoterodine fumarate.

### Background of the Invention

Fesoterodine fumarate is the fumarate salt form of fesoterodine, a competitive muscarinic receptor antagonist with muscle relaxant and urinary antispasmodic properties. Fesoterodine is rapidly hydrolyzed in vivo into its active metabolite 5-hydroxy methyl tolterodine, which binds and inhibits muscarinic receptors on the bladder detrusor muscle, thereby preventing bladder contractions or spasms caused by acetylcholine. This results in the relaxation of bladder smooth muscle and greater bladder capacity, in addition to a reduction in involuntary muscle contractions and involuntary loss of urine. The active metabolite does not interact with alpha-adrenergic, serotonergic, histaminergic and excitatory amino acid receptors and is eliminated via renal excretion.

The chemical name of fesoterodine fumarate is (E)-but-2-enedioic acid;[2-[(1R)-3-[di(propan-2-yl)amino]-1-phenylpropyl]-4-(hydroxymethyl)phenyl] 2-methylpropanoate. Its empirical formula is C₃₀H₄₁NO₇ and has the following structural Formula I:

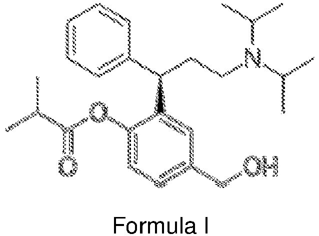

The drug fesoterodine fumarate is the active ingredient in a product being sold as TOVIAZ^{®} tablets to treat urinary incontinence and frequency problems. Inactive ingredients are glyceryl behenate, hypromellose, lactose monohydrate, soya lecithin, microcrystalline cellulose, polyethylene glycol, polyvinyl alcohol, talc, titanium dioxide, and xylitol.

Fesoterodine fumarate is known in the art for its potency in treating urinary incontinence.

However, fesoterodine may exhibit substantial degradation under stress conditions. It is believed that hydrolyzation and oxidation are among the major mechanisms resulting in degradation.

In the prior art, there are also several patents which disclose an extended release tablet composition comprising fesoterodine mostly in oral pharmaceutical dosage forms. However, these compositions have some problems.

US2015182629 patent application discloses a pharmaceutical composition comprising fesoterodine fumarate. In order to ensure stability, the application had not been mentioned the importance of the coating and any coating agents.

WO2010043408 discloses microencapsulated fesoterodine; wherein fesoterodine is present in a core which is surrounded by shell; the shell contains excipients which modify the release of fesoterodine. The microencapsulated fesoterodine is further formulated in the form of tablets. But, the described process in the application is complex, time consuming and costs are high.

WO2014138603 discloses fesoterodine fumarate tablets without a coating of ammonium methacrylate copolymer.

There is thus still a need for a physically and chemically stable composition comprising fesoterodine that are stable against fesoterodine degradation over an extended period of time.

In the present invention, the certain coating agents were used and so while the stable formulation is provided so, the formulation provides desired stability, desired dissolution profile and excellent hardness. Also, the formulation has been developed by using cost-effective and time-saving process which is a simple method according to the prior art.

### Detailed Description of the Invention

The main object of the present invention is to provide an extended release tablet composition comprising fesoterodine fumarate which provides desired stability, dissolution profile and excellent hardness by using the improved coating.

Another object of the present invention is to provide an extended release tablet composition comprising fesoterodine fumarate which is more stable against degradation over storage period and also provide desired extended release of the drug.

The term "extended release" may be defined as reaching desired plasma levels of an active agent of interest throughout a determined period of time and providing the drug release at a uniform and constant rate. Fesoterodine fumarate is highly soluble in water. High solubility affects the dissolution profile and it may cause dose dumping which is a result of too rapid release of the active agent. In this present invention, to provide an extended release and to control the release rate, release retarding agents have been used.

According to the present invention, the extended release composition comprising fesoterodine fumarate is in the dosage form of tablet. Tablet form is selected from the group comprising bilayer tablet, multilayer tablet, mini tablet, intraoral tablet, sublingual tablet, effervescent tablet, rapid release tablets, intra-tablet tablet, inlay tablet, tablet in tablet, film-coated tablet or enteric-coated tablet.

According to the present invention, the extended release tablet composition comprises fesoterodine fumarate and at least one coating comprising coating agents wherein the amount of fesoterodine fumarate is between 0.5% and 25.0% w/w of the composition and the amount of the coating agents is between 0.1% and 10.0% w/w of the composition.

According to another embodiment of this present invention, said coating is film coating.

In one embodiment of this present invention, the amount of fesoterodine fumarate is between 0.5% and 15.0% w/w, preferably between 1.0% and 8.0% w/w of the composition.

According to the present invention, the extended release tablet composition is coated with suitable coating agents so that fesoterodine fumarate was not affected by stress conditions such as heat, humidity.

Especially, used ammonium methacrylate copolymer mixture as a coating agent ensure protection of the tablet from stress conditions such as heat, humidity. So, tablet compositions are more stable against degradation over an extended period of time even under stress conditions.

According to the present invention, the coating agents are ammonium methacrylate copolymer mixture, triethyl citrate, talc, silicon dioxide.

In one embodiment of the present invention, coating the extended release tablet composition comprises;

The coating comprises;
- 0.1% to 5.0% by weight of ammonium methacrylate copolymer mixture
- 0.1% to 1.5% by weight of triethyl citrate
- 0.1% to 3.0% by weight of talc
- 0.1% to 1.5% by weight of silicon dioxide of the composition.

According to another embodiment of this present invention, the extended release tablet composition further comprises at least one pharmaceutically acceptable excipient which is selected from the group comprising release retarding agents, stabilizers, diluents, lubricants or the mixtures thereof.

According to this embodiment, the polymer as release retarding agent is selected from the group comprising hydroxyl propyl methylcellulose (HPMC) such as HPMC E4M and HPMC K100, hydroxyethyl methylcellulose, hydroxypropyl cellulose, hydroxypropyl ethylcellulose, ethylcellulose, methacrylic acid - ethyl acrylate copolymer, polymethylmetacrylate or copolymers, polyvinyl acetate, polyvinyl alcohol, polyvinylpyrrolidine, polyethylene oxide, polyethylene glycol, cellulose acetate, vinyl acetate/crotonic acid copolymers, maleic anhydride/methyl vinyl ether copolymers, copolymer of acrylic or methacrylic acid esters, polyoxyethylene -alkyl ethers, or mixtures thereof.

Preferably the polymer release retarding agents is hydroxy propyl methylcellulose.

In one embodiment of this present invention, the amount of release retarding agent is between 1.0 % and 60.0 % w/w, preferably 3.0 % and 55.0 % w/w, more preferably 5.0 % and 50.0 w/w, more preferably 9.0 % and 43.0 w/w of the composition.

According to this embodiment, the ratio of fesoterodine fumarate to the release retarding agent is in the range of between 0.1:7 and 7:0.1 by weight, preferably between 0.1:5 and 5:0.1 by weight, more preferably 0.1:4 and 4:0.1 by weight of the composition.

According to the present invention, the extended release tablet composition comprises at least one sugar as stabilizer, which is fructose.

In the present invention, the extended release tablet composition comprises fructose or fructose-sucrose mixture as stabilizer.

According to one embodiment of the present invention, the fructose-sucrose mixture is a stabilizer which also has binder properties. In addition, it further enhances excellent pharmacotechnical properties (i.e. flowability, compressibility and homogeneity).

In one embodiment of this present invention, the amount of the fructose-sucrose mixture is between 1.0% and 40.0% by weight of the composition, preferably it is between 5.0% and 35.0% by weight, more preferably it is between 10.0% and 30.0% by weight of the composition.

Suitable diluents are selected from the group lactose monohydrate, microcrystalline cellulose, calcium carbonate, calcium phosphate dibasic, calcium phosphate tribasic, calcium sulfate, microcrystalline silicified cellulose, powdered cellulose, dextrates, dextrose, fructose, lactitol, lactose anhydrous, lactose dihydrate or mixtures thereof.

In one embodiment of this present invention, the extended release tablet composition comprises lactose monohydrate, microcrystalline cellulose mixture as a diluent.

According to another embodiment of this present invention, the amounts of diluents are between 10.0% and 50.0% w/w of the composition, preferably the amounts of diluents are between 15.0% and 45.0% w/w of the composition.

Suitable lubricants are selected from the group from talc, calcium silicate, powdered cellulose, starch, colloidal silicon dioxide or mixtures thereof.

In one embodiment of this present invention, the extended release composition comprises talc as a lubricant.

According to another embodiment of this present invention, the lubricants are between 0.1% and 5.0% w/w of the composition.

In one embodiment of the present invention, the extended release tablet composition comprises;
- 0.5 % - 25.0 % fesoterodine fumarate
- 0.1 % - 10.0 % coating agents which are ammonium methacrylate copolymer mixture, triethyl citrate, talc, silicon dioxide.

In one embodiment of the invention, the extended release tablet composition comprises;
0.50 % - 25.0 % by weight of fesoterodine fumarate
0.90 % - 35.0 % by weight of fructose
0.10 % - 5.00 % by weight of sucrose
2.50 % - 25.0 % by weight of lactose monohydrate
2.50 % - 25.0 % by weight of microcrystalline cellulose
0.20 % - 5.00 % by weight of glyceryl dibehenate
0.40 % - 23.0 % by weight of hydroxypropyl methylcellulose
0.10 % - 5.00 % by weight of talc
0.1 % - 10.0 % by weight of coating agents which are ammonium methacrylate copolymer mixture, triethyl citrate, talc, silicon dioxide.

The extended release tablet composition of the present invention may be prepared by direct compression, wet or dry granulation, hot melt granulation, hot melt extrusion, fluidized bed granulation, extrusion/spheronization, slugging, spray drying or solvent evaporation.

Process for preparing the extended release tablet composition comprises the following steps;
a) Mixing fesoterodine fumarate with fructose and sucrose
b) Granulating the mixture with water then, sieving
c) Drying the mixture at 40°C until the humidity is less than 0.5%, then sieving the mixture
d) Adding lactose monohydrate, microcrystalline cellulose, glyceryl dibehenate, hydroxypropyl methylcellulose and then mixing
e) Then, adding talc and mixing
f) Then, pressing to form tablet
g) Coating tablets with coating agent which are ammonium methacrylate copolymer mixture, triethyl citrate, talc, silicon dioxide.

It has been found that using wet granulation process, granulating fesoterodine fumarate with the addition of excipients for wet granulation showed an improved stability of fesoterodine fumarate in the granulate and in the final pharmaceutical composition.

Also, it has been found that when the composition is prepared with using water-based granulation, resulting compositions possess improved stability.

The given below examples describes the extended release tablet composition comprising fesoterodine.

### Example 1: Extended release tablet composition comprising fesoterodine

| **Agents** | **Amount (% by weight of the total composition)** |
|---|---|
| Fesoterodine fumarate | 0.5% to 25.0% |
| Fructose | 0.9 % - 35.0% |
| Sucrose | 0.1 % - 5.0% |
| Lactose monohydrate | 2.5 % - 25.0% |
| Microcrystalline cellulose | 2.5 % - 25.0% |
| Glyceryl dibehenate | 0.2 % - 5.0% |
| Hydroxypropyl methylcellulose | 0.4 % - 23.0% |
| Talc | 0.1 % - 5.0% |
| Coating | 0.1 %- 10.0% |
| **Total** | **100** |

### * Coating comprising:

| | **Amount (% by weight of the total composition)** |
|---|---|
| Ammonium methacrylate copolymer mixture | 0.1% to 5.0% |
| Talc | 0.1% to 3.0% |
| Triethyl citrate | 0.1% to 1.5% |
| Silicon dioxide | 0.1% to 1.5% |

### Process for example 1:

The process for preparation of the modified release composition comprises the following steps:
h) Mixing fesoterodine fumarate with fructose and sucrose
i) Granulating the mixture with water then, sieving
j) Drying the mixture at 40°C until the humidity is less than 0.5%, then sieving the mixture
k) Adding lactose monohydrate, microcrystalline cellulose, glyceryl dibehenate, hydroxypropyl methylcellulose and then mixing
l) Then, adding talc and mixing
m) Then, pressing to form tablet
n) Coating tablets with coating agents which are ammonium methacrylate copolymer mixture, triethyl citrate, talc, silicon dioxide.

## Claims

1. An extended release tablet composition comprising fesoterodine fumarate and at least one coating comprising coating agents wherein the amount of fesoterodine fumarate is between 0.5% and 25.0% w/w of the composition and the amount of the coating agents is between 0.1% and 10.0% w/w of the composition and wherein the coating agents are ammonium methacrylate copolymer mixture, triethyl citrate, talc and silicon dioxide; and wherein the extended release tablet composition further comprises fructose as a stabilizer.

2. The extended release tablet composition according to claim 1, wherein the amount of fesoterodine fumarate is between 0.5% and 15.0% w/w, preferably between 1.0% and 8.0% w/w of the composition.

3. The extended release tablet composition according to claim 1, wherein said coating is film coating.

4. The extended release tablet composition according to claim 1, further comprises at least one pharmaceutically acceptable excipient which is selected from the group comprising release retarding agents, stabilizers, diluents, lubricants or the mixtures thereof.

5. The extended release tablet composition according to claim 4, wherein at least one polymer as the release retarding agent is selected from the group comprising hydroxyl propyl methylcellulose, hydroxyethyl methylcellulose, hydroxypropyl cellulose, hydroxypropyl ethylcellulose, ethylcellulose, methacrylic acid - ethyl acrylate copolymer, polymethylmetacrylate or copolymers, polyvinyl acetate, polyvinyl alcohol, polyvinylpyrrolidine, polyethylene oxide, polyethylene glycol, cellulose acetate, vinyl acetate/crotonic acid copolymers, maleic anhydride/methyl vinyl ether copolymers, copolymer of acrylic or methacrylic acid esters, polyoxyethylene-alkyl ethers, or mixtures thereof.

6. The extended release tablet composition according to claim 5, wherein the amount of release retarding agent is between 1.0 % and 60.0 %, preferably 3.0 % and 55.0 %, more preferably 5.0 % and 50.0 w/w of the composition.

7. The extended release tablet composition according to claim 4, wherein the diluents are selected from the group lactose monohydrate, microcrystalline cellulose, calcium carbonate, calcium phosphate dibasic, calcium phosphate tribasic, calcium sulfate, microcrystalline silicified cellulose, powdered cellulose, dextrates, dextrose, fructose, lactitol. lactose anhydrous, lactose dihydrate or mixtures thereof.

8. The extended release tablet composition according to any preceding claims, the composition comprising;
0.50 % - 25.0 % by weight of fesoterodine fumarate
0.90 % - 35.0 % by weight of fructose
0.10 % - 5.00 % by weight of sucrose
2.50 % - 25.0 % by weight of lactose monohydrate
2.50 % - 25.0 % by weight of microcrystalline cellulose
0.20 % - 5.00 % by weight of glyceryl dibehenate
0.40 % - 23.0 % by weight of hydroxypropyl methylcellulose
0.10 % - 5.00 % by weight of talc
0.1 % - 10.0 % by weight of coating agents which are ammonium methacrylate copolymer mixture, triethyl citrate, talc, silicon dioxide.

9. A process for preparing the modified release tablet composition according to claim 8, the composition comprising the following steps;
a) Mixing fesoterodine fumarate with fructose and sucrose
b) Granulating the mixture with water then, sieving
c) Drying the mixture at 40°C until the humidity is less than 0.5%, then sieving the mixture
d) Adding lactose monohydrate, microcrystalline cellulose, glyceryl dibehenate, hydroxypropyl methylcellulose and then mixing
e) Then, adding talc and mixing
f) Then, pressing to form tablet
g) Coating tablets with coating agent which are ammonium methacrylate copolymer mixture, triethyl citrate, talc, silicon dioxide.

## Patentansprüche

1. Zusammensetzung einer Tablette für die Langzeitfreisetzung, die Fesoterodinfumarat und wenigstens eine Beschichtung mit Beschichtungsmittel aufweist, wobei die Menge an Fesoterodinfumarat zwischen 0,5 % und 25,0 % (m/m) der Zusammensetzung liegt und die Menge an Beschichtungsmittel zwischen 0,1 % und 10,0 % (m/m) der Zusammensetzung liegt und wobei die Beschichtungsmittel eine Ammoniummethacrylat-Copolymer-Gemisch, Triethylcitrat, Talk und Siliciumdioxid sind; und wobei die Zusammensetzung der Tablette für die Langzeitfreisetzung ferner Fructose als Stabilisator aufweist.

2. Zusammensetzung einer Tablette für die Langzeitfreisetzung nach Anspruch 1, wobei die Menge an Fesoterodinfumarat zwischen 0,5 % und 15,0 % (m/m), vorzugsweise zwischen 1,0 % und 8,0 % (m/m) der Zusammensetzung beträgt.

3. Zusammensetzung einer Tablette für die Langzeitfreisetzung nach Anspruch 1, wobei die Beschichtung eine Filmbeschichtung ist.

4. Zusammensetzung einer Tablette für die Langzeitfreisetzung nach Anspruch 1, die ferner wenigstens einen pharmazeutisch verträglichen Hilfsstoff aufweist, der aus der Gruppe ausgewählt ist, die freisetzungsverzögernde Mittel, Stabilisatoren, Verdünnungsmittel, Gleitmittel oder deren Mischungen umfasst.

5. Zusammensetzung einer Tablette für die Langzeitfreisetzung nach Anspruch 4, wobei als das freisetzungsverzögernde Mittel wenigstens ein Polymer ausgewählt ist aus der Gruppe, die Hydroxylpropylmethylcellulose, Hydroxyethylmethylcellulose, Hydroxypropylethylcellulose, Ethylcellulose, Methacrylsäure-Ethylacrylat-Copolymer aufweist, Polymethylmetacrylat oder Copolymere, Polyvinylacetat, Polyvinylalkohol, Polyvinylpyrrolidin, Polyethylenoxid, Polyethylenglykol, Celluloseacetat, Copolymere aus Vinylacetat und Crotonsäure, Copolymere aus Maleinsäureanhydrid und Methylvinylether, Copolymere aus Acrylsäure- oder Methacrylsäureestern, Polyoxyethylenalkylether oder Mischungen davon.

6. Zusammensetzung einer Tablette für die Langzeitfreisetzung nach Anspruch 5, wobei die Menge des freisetzungsverzögernden Mittels zwischen 1,0 % und 60,0 %, vorzugsweise zwischen 3,0 % und 55,0 %, noch bevorzugter zwischen 5,0 % und 50,0 % (m/m) der Zusammensetzung liegt.

7. Zusammensetzung einer Tablette für die Langzeitfreisetzung nach Anspruch 4, wobei die Verdünnungsmittel aus der Gruppe Lactosemonohydrat, mikrokristalline Cellulose, Calciumcarbonat, dibasisches Calciumphosphat, dreibasisches Calciumphosphat, Calciumsulfat, mikrokristalline verkieselte Cellulose, pulverisierte Cellulose, Dextrate, Dextrose, Fructose, Lactitol, wasserfreie Lactose, Lactosedihydrat oder Mischungen davon ausgewählt sind.

8. Zusammensetzung einer Tablette für die Langzeitfreisetzung nach einem der vorhergehenden Ansprüche, wobei die Zusammensetzung folgendes aufweist: 0,50 % - 25,0 Gew.-% Fesoterodinfumarat
0,90 - 35,0 Gew.-% Fructose
0,10 - 5,00 Gew.-% Saccharose
2,50 - 25,0 Gew.-% Lactose-Monohydrat
2,50 - 25,0 Gew.-% mikrokristalline Cellulose
0,20 - 5,00 Gew.-% Glyceryldibehenat
0,40 - 23,0 Gewichtsprozent Hydroxypropylmethylcellulose
0,10 - 5,00 Gew.-% Talkum
0,1 - 10,0 Gew.-% Beschichtungsmittel, die Ammoniummethacrylat-Copolymer-Gemisch, Triethylcitrat, Talkum, Siliciumdioxid sind.

9. Verfahren zur Herstellung der Tablettenzusammensetzung mit modifizierter Freisetzung gemäß Anspruch 8, wobei die Zusammensetzung die folgenden Schritte aufweist;
a) Mischen von Fesoterodinfumarat mit Fructose und Saccharose
b) Granulieren der Mischung mit Wasser und anschließendes Sieben
c) Trocknen der Mischung bei 40°C, bis die Feuchtigkeit weniger als 0,5% beträgt, dann Sieben der Mischung
d) Zugabe von Laktosemonohydrat, mikrokristalliner Cellulose, Glycerindibehenat und Hydroxypropylmethylcellulose und anschließendes Mischen
e) Dann Talkum hinzufügen und mischen
f) Ausbilden einer Tablette durch Pressen
g) Beschichtung der Tabletten mit einem Beschichtungsmittel, das Ammoniummethacrylat-Copolymer-Gemisch, Triethylcitrat, Talkum und/oder Siliciumdioxid ist.

## Revendications

1. - Composition de comprimé à libération prolongée, comprenant du fumarate de fésotérodine et au moins un enrobage comprenant des agents d'enrobage, composition dans laquelle la quantité de fumarate de fésotérodine est entre 0,5 % et 25,0 % p/p de la composition et la quantité des agents d'enrobage est entre 0,1 % et 10,0 % p/p de la composition et dans laquelle les agents d'enrobage sont un mélange copolymère de méthacrylate d'ammonium, du citrate de triéthyle, du talc et du dioxyde de silicium ; et dans laquelle la composition de comprimé à libération prolongée comprend en outre du fructose en tant que stabilisant.

2. - Composition de comprimé à libération prolongée selon la revendication 1, dans laquelle la quantité de fumarate de fésotérodine est entre 0,5 % et 15,0 % p/p, de préférence entre 1,0 % et 8,0 % p/p de la composition.

3. - Composition de comprimé à libération prolongée selon la revendication 1, dans laquelle l'enrobage est un pelliculage.

4. - Composition de comprimé à libération prolongée selon la revendication 1, comprenant en outre au moins un excipient pharmaceutiquement acceptable qui est choisi dans le groupe comprenant les agents retardateurs de libération, les stabilisants, les diluants, les lubrifiants ou les mélanges de ceux-ci.

5. - Composition de comprimé à libération prolongée selon la revendication 4, dans laquelle au moins un polymère comme agent retardateur de libération est choisi dans le groupe comprenant l'hydroxyl propyl méthylcellulose, l'hydroxyéthyl méthylcellulose, l'hydroxypropyl cellulose, l'hydroxypropyl éthylcellulose, l'éthylcellulose, un copolymère acide méthacrylique - acrylate d'éthyle, le poly(méthacrylate de méthyle) ou ses copolymères, le poly(acétate de vinyle), l'alcool polyvinylique, la polyvinylpyrrolidine, le poly(oxyde d'éthylène), le polyéthylène glycol, l'acétate de cellulose, les copolymères acétate de vinyle / acide crotonique, les copolymères anhydride maléique / méthyl vinyl éther, les copolymères d'esters d'acide acrylique ou méthacrylique, les polyoxyéthylène-alkyl éthers ou les mélanges de ceux-ci.

6. - Composition de comprimé à libération prolongée selon la revendication 5, dans laquelle la quantité d'agent retardateur de libération est entre 1,0 % et 60,0 %, de préférence entre 3,0 % et 55,0 %, de façon davantage préférée entre 5,0 % et 50,0 % p/p de la composition.

7. - Composition de comprimé à libération prolongée selon la revendication 4, dans laquelle les diluants sont choisis dans le groupe comprenant le lactose monohydraté, la cellulose microcristalline, le carbonate de calcium, le phosphate de calcium dibasique, le phosphate de calcium tribasique, le sulfate de calcium, la cellulose microcristalline silicifiée, la cellulose en poudre, les dextrates, le dextrose, le fructose, le lactitol, le lactose anhydre, le lactose dihydraté ou les mélanges de ceux-ci.

8. - Composition de comprimé à libération prolongée selon l'une quelconque des revendications précédentes, la composition comprenant :
0,50 % à 25,0 % en poids de fumarate de fésotérodine
0,90 % à 35,0 % en poids de fructose
0,10 % à 5,00 % en poids de saccharose
2,50 % à 25,0 % en poids de lactose monohydraté
2,50 % à 25,0 % en poids de cellulose microcristalline
0,20 % à 5,00 % en poids de dibéhénate de glycéryle
0,40 % à 23,0 % en poids d'hydroxypropyl méthylcellulose
0,10 % à 5,00 % en poids de talc
0,1 % à 10,0 % en poids d'agents d'enrobage qui sont un mélange copolymère de méthacrylate d'ammonium, du citrate de triéthyle, du talc, du dioxyde de silicium.

9. - Procédé de préparation de la composition de comprimé à libération modifiée selon la revendication 8, la composition comprenant les étapes suivantes ;
a) mélanger le fumarate de fésotérodine avec le fructose et le saccharose
b) granuler le mélange avec de l'eau, puis tamiser
c) sécher le mélange à 40°C jusqu'à ce que l'humidité soit inférieure à 0,5 %, puis tamiser le mélange
d) ajouter le lactose monohydraté, la cellulose microcristalline, le dibéhénate de glycéryle , l'hydroxypropyl méthylcellulose, puis mélanger
e) puis, ajouter le talc et mélanger
f) puis, presser pour former un comprimé
g) enrober les comprimés par des agents d'enrobage qui sont un mélange copolymère de méthacrylate d'ammonium, du citrate de triéthyle, du talc, du dioxyde de silicium.
